Europäisches Patentamt

European Patent Office

Office européen des brevets

(11) Veröffentlichungsnummer: **0 303 262**
**A2**

# (12) EUROPÄISCHE PATENTANMELDUNG

(21) Anmeldenummer: 88113035.5

(22) Anmeldetag: 11.08.88

(51) Int. Cl.⁴: **C12N 11/14**

(30) Priorität: **13.08.87 DE 3726941**
**13.08.87 DE 3726942**

(43) Veröffentlichungstag der Anmeldung:
**15.02.89 Patentblatt 89/07**

(84) Benannte Vertragsstaaten:
**AT BE CH DE ES FR GB GR IT LI LU NL SE**

(71) Anmelder: **Kernforschungsanlage Jülich**
**Gesellschaft mit beschränkter Haftung**
**Postfach 1913**
**D-5170 Jülich 1(DE)**

(84) **BE CH DE ES FR GR IT LI LU NL SE AT**

(71) Anmelder: **Schott Glaswerke**
**Hattenbergstrasse 10**
**D-6500 Mainz(DE)**

(84) **BE CH DE ES FR GR IT LI LU NL SE AT**

(71) Anmelder: **Carl-Zeiss-Stiftung trading as**
**SCHOTT GLASWERKE**
**Hattenbergstrasse 10**
**D-6500 Mainz 1(DE)**

(84) **GB**

(72) Erfinder: **Büchs, Jochen**
**Am Klehf 9**
**D-2071 Rotenbek(DE)**
Erfinder: **Wandrey, Christian, Prof.**
**Wolfshovener Strasse 139**
**D-5170 Jülich(DE)**
Erfinder: **Kula, Maria-Regina, Prof.**
**Seigenbusch 12**
**D-5172 Niederzier-Hambach(DE)**
Erfinder: **Radke, Manfred**
**Im Rell 2**
**D-6501 Heidesheim-Heidenfahrt(DE)**

(54) **Anorganische Trägerkörper mit aminhaltiger Oberflächenschicht zur Immobilisierung von Mikroorganismen oder Zellen, Verfahren zur Herstellung derselben und deren Verwendung.**

(57) Anorganische Trägerkörper, insbesondere aus Glas oder Keramik für die Immobilisierung von Mikroorganismen oder von menschlichen, tierischen oder pflanzlichen Zellen werden mit einer die Zellanlagerung fördernden Oberflächenschicht versehen, die durch Dialkylaminoalkyldextran, insbesondere DEAE-Dextran gebildet wird, das rein adsorbtiv oder kovalent an den Träger gebunden ist. Bevorzugt werden DEAE-Dextrane mit einem möglichst geringen Anteil an dimeren DEAE-Gruppen, der insbesondere < 50 %, insbesondere ≤ 35 % ist. Molekulargewichte zwischen 100.000 und 2 x 10⁶ sind zweckmäßig, die möglichst groß sein sollen, aber noch nicht zu einem Kneulen führen. Der N-Gehalt liegt vorzugsweise zwischen 0,5 und 5 %, insbesondere 3 - 4 % und vorzugsweise bei 3,3 %. Mit einer solchen Oberflächenschicht werden insbesondere offenporige Sintergläser mit einer Porendoppelstruktur

versehen, die Makroporen von 20 bis 500 μm aufweisen sowie in den Makroporenwänden Mikroporen
von 1 bis 10 μm haben.

**Glas behandelt mit**
- △——△ DEAE - Dextran
- ⊟---⊟ Chitosan
- ◇-·-◇ Eudragit RL
- ✳-·-✳ Eudragit E
- ○——○ unbehandeltes Glas

FIG. 1

y-axis: m Mol/l Leucin
x-axis: h

## Anorganische Trägerkörper mit aminhaltiger Oberflächenschicht zur Immobilisierung von Mikroorganismen oder Zellen, Verfahren zur Herstellung derselben und deren Verwendung

Die Erfindung bezieht sich auf anorganische Trägerkörper, insbesondere aus Glas oder Keramik, mit basischer, aminhaltiger Oberflächenschicht zur Immobilisierung von Mikroorganismen oder von menschlichen, tierischen oder pflanzlichen Zellen sowie auf deren Herstellung und Verwendung.

Die Immobilisierung von Mikroorganismen spielt seit langer Zeit eine erhebliche Rolle bei der Durchführung biotechnologischer Prozesse, da auf diese Weise lokal begrenzt erhebliche Konzentrationen an biologisch wirksamer Masse zur Verfügung gestellt werden können, von denen die im allgemeinen flüssige Reaktionsmischung relativ leicht abtrennbar ist.

So gehören organische Trägermaterialien, wie Buchenholzspäne, Stroh, Sägemehl, Alfalfa-Häcksel usw., seit langem zum Stande der Tecknik ebenso wie anorganische Materialien in Form von Glasperlen, Splitt, Steingut, Bimsstein, Lava, Sand u.dgl. Man kennt auch bereits synthetische Trägermaterialien aus Carboxymethylcellulose, Polyurethanschaum, Äthylen-Maleinsäureanhydrid-Copolymeren, Cellulosetriacetat u.dgl., die eine relativ große Oberfläche besitzen.

Es ist auch seit längerer Zeit bekannt, Glasoberflächen (insbesondere von porösen Membranen) durch Polymerfilme oder durch Umsetzung der Silanolgruppen mit funktionelle Gruppen aufweisenden organischen Molekülen zu verändern (DE-PS 24 54 111).

Für chromatographische Zwecke sind Glasträger bekannt, die mit ggf. vernetztem Polysaccharid beschichtet sind, das durch aufgepfropfte Moleküle oder Makromoleküle mit Aminogruppen modifiziert ist (FR-A1-2 422 699; als spezielles Beispiel wird hier mikroporöses Glas mit einer Schicht aus Cellulose oder vernetztem DEAE-Dextran genannt, die durch aufpfropfte biospezifische Verbindungen modifiziert sind) oder auch Glasträger mit chemisch gebundenem Polyethylenimin (J. Chromat. 120 (1976), 321 - 333).

Solche Techniken der Veränderung von porösen Glasoberflächen wurden auch bereits für Trägerkörper für Mikroorganismen vorgesehen: So werden in der PCT-Anmeldung WO 87/02703 sog. Microcarriers aus porösem Glas beschrieben, die mit Amino- oder Hydroxylgruppen aufweisenden Polymeren beschichtet sind. Aus der Vielzahl der angegebenen Möglichkeiten werden dabei Chitosan, Cellulose, Alginsäure und nachträglich vernetzte Überzüge aus Agar, Gelatine oder K-Karragheenan hervorgehoben.

Für die Immobilisierung von Zellen und Mikroorganismen wurde gemäß der US-PS 4,153,510 auch eine chemische Kopplung der Zellen selbst vorgesehen.

Ferner sind aus der US-PS 4, 189,534 Microcarriers für Zellkulturen bekannt, die aus vernetztem DEAE- Dextran bestehen. Über die Anlagerung von Zellen an DEAE-Sephadex wurde auch bereits von van Welzel in Nature 216 (1967), 64 - 65, berichtet.

In einer Arbeit von C.D. Buck u.a. (J. Virol. Methods 10 (1985), 171 - 184) wird allerdings über einen Vergleich von Microcarriers aus Polyacrylamid, Polystyrol und vernetztem DEAE-Dextran berichtet, bei dem ein ungenügendes Verhalten der Microcarriers aus vernetztem DEAE-Dextran gefunden wurde.

Es wurde nun überraschenderweise festgestellt, daß durch Beschichtung von Glas mit nichtvernetztem, Dialkylaminoalkyl-Dextran, insbesondere DEAE-Dextran, eine besonders gute Immobilisierung von Mikroorganismen auf anorganischen Trägerkörpern, insbesondere Glas, erzielt werden kann. Die erfindungsgemäßen Trägerkörper der eingangs genannten Art sind demgemäß dadurch gekennzeichnet, daß die Oberflächenschicht durch Dialkylaminoalkyl-Dextran, insbesondere DEAE-Dextran, gebildet wird und an den anorganischen Trägerkörper adsorptiv oder insbesondere kovalent gebunden ist.

Solche mit adsorptiv oder insbesondere kovalent gebundenen Oberflächenschichten aus nichtvernetztem Dialkylaminoalkyl-Dextran versehenen Träger zeigen ein deutlich verbessertes Anlagerungsvermögen für Mikroorganismen und Zellen und sind somit im Rahmen biotechnologischer Prozesse besonders günstig anwendbar.

Die zu beschichtenden Trägerkörper sind insbesondere oxidische oder silikatische Körper z.B. aus Glas, Quarz, Aluminiumoxid, Zirkonoxid usw., wobei Glas wegen der relativ einfachen Erzielung definierter Körper bevorzugt wird.

Die zu beschichtenden Körper können als Membranen, Fasern bzw. Fäden, Rohre, Kapillaren, Microcarriers oder insbesondere poröse Trägerkörper von beliebiger Gestalt vorliegen.

Besonders bevorzugt werden poröse Trägerkörper mit der in der DE-OS 34 10 650 beschriebenen, speziellen Porenstruktur, die porositätsbestimmende durchgehende Makroporen (von insbesondere 20 - 500 $\mu$m) für einen ungehemmten Flüssigkeits- und Gasaustausch vom Trägerinneren zur Umgebung hin sowie die Makroporenwände durchsetzende Mikroporen (von insbesondere 1 - 10 $\mu$m) umfassen, deren Größe im Bereich der Größe der Mikroorganismen oder Zellen liegen soll.

Die gemäß der Erfindung beschichteten Trägerkörper können unbegrenzt autoklaviert werden. Sie sind insbesondere für die sterile Kultivierung von Mikroorganismen bzw. Zellen geeignet, die auf üblichen unbehandelten Glas- oder Keramikoberflächen nur schlecht haften bzw. aufwachsen. Besonders geeignet sind beschichtete poröse Trägerkörper wegen ihrer Formstabilität und hohen Dichte für Festbett- und Wirbelschichtreaktoren, in denen aerobe oder anaerobe Prozesse mit hoher Gasentwicklung oder Prozesse mit der Gefahr von Transportlimitierung ablaufen, wobei dann insbesondere Trägerkörper mit hoher Offenporigkeit verwendet werden.

Besonders untersucht wurden Trägerkörper aus Glas mit DEAE-Dextran-Überzug. Besonders bevorzugt werden danach DEAE-Dextrane mit einem hohen Stickstoffgehalt, aber relativ geringem Anteil an dimeren DEAE-Gruppen und einem speziellen Größenbereich der Molekulargewichte verwendet:

Da einfache DEAE-Gruppen einen pK-Wert von 9,2, dimere DEAE-Gruppen jedoch einen solchen von ca. 6,3 haben, sind die dimeren Gruppen bei den im biotechnologischen Bereich weitgehend üblichen Betriebsbedingungen (um pH 7) nur zum kleinen Teil dissoziiert und daher weniger nützlich. Ihr Anteil sollte vorzugsweise unter 50%, insbesondere nicht über 35 % liegen.

Das Molekulargewicht des für die Beschichtung verwendeten DEAE-Dextrans sollte etwa zwischen 100 000 und 2 000 000 liegen, vorzugsweise $\leq 10^6$ sein, wobei jedoch in den oberen Bereichen eine Faltung der sonst gestreckten DEAE-Dextran-Moleküle beginnt, die nicht erwünscht ist. Molekulargewichte um 600 000 werden daher bevorzugt.

Der Stickstoffgehalt sollte vorzugsweise zwischen 0,5 und 5 %, insbesondere um 3 bis 4 %, liegen, wobei mit einem Material mit 3,3 % N-Gehalt gute Ergebnisse erzielt wurden.

Vorzugsweise werden diese haftvermittelnden Schichten kovalent mit dem anorganischen Körper gekoppelt und sind dann beständig auf dem Träger aufgezogen, der unterschiedlichen Bedingungen ausgesetzt werden kann, ohne daß eine Ablösung der haftvermittelnden Schicht zu befürchten wäre. Insbesondere werden dadurch Reinigung und Regenerierung des Trägermaterials zwischen zwei biotechnologischen Anwendungen unproblematisch.

Für eine solche Bindung des Dialkylaminoalkyldextrans an Glasträger eignen sich solche mit Glycidyloxyalkylglas (Epoxyalkylglas)- oder Chloralkylglasoberfläche oder auch mit Amonialkylglasoberfläche, an welch letztere eine Kopplung mit Hilfe von Diisocyanaten erfolgt.

An Hydroxylgruppen enthaltende Träger können Dialkylaminoalkyldextrane in analoger Weise gebunden werden.

Die Alkylgruppen des Dialkylaminoalkyldextrans können gleich oder verschieden sein und bedeuten insbesondere niedere Alkylgruppen mit 1 bis 4 Kohlenstoffatomen, spezielle 1 oder 2 Kohlenstoffatomen. Die vorliegenden Untersuchungen wurden weitgehend mit Diethylaminoethyldextran (DEAE-Dextran) durchgeführt, über das daher vornehmlich berichtet wird.

Die praktische Durchführung der Kopplungsreaktionen bietet keine grundsätzlichen Besonderheiten. Als Beispiel wird nachfolgend die Bindung von DEAE-Dextran an Glycidylglas in Dimetylsulfoxid beschrieben.

110 g vier Stunden in 5%iger $HNO_3$ gekochtes, gespültes und getrocknetes Sinterglasmaterial gemäß DE-OS 34 10 650 wurden zu 10 ml gamma-Glycidyloxypropyltrimethoxysilan und 490 ml wasserfreiem Aceton gegeben, durch Anlegen von Vakuum entgast und einen Tag bei Raumtemperatur belassen. Anschließend wurde das material dreimal mit wasserfreiem Aceton gewaschen und dann zwei Stunden bei 60°C und einen Tag bei Raumtemperatur getrocknet. Danach wurde das so behandelte Sinterglas in eine 3%ige Lösung von DEAE-Dextran in wasserfreiem Dimethylsulfoxid gegeben, durch Vakuum entgast, drei Tage bei Raumtemperatur und dann einen Tag bei 60°C geschüttelt, anschließend gewaschen und getrocknet.

Als Lösungsmittel kann auch Wasser an Stelle von Dimethylsulfoxid verwendet werden.

Für die Erzeugung von einfachen Adsorptionsschichten auf porösen Glasträgern werden diese vorzugsweise in eine wäßrige DEAE-Dextran-Lösung, insbesondere in eine etwa 3%ige DEAE-Dextran-Lösung, gegeben, und durch Anlegen von Unterdruck wird für ein gutes Vordringen der Lösung zu den inneren Oberflächen der Trägerkörper gesorgt.

Diese werden vorzugsweise vorher mit starker Säure behandelt, wobei sich ein längeres Kochen in Salpetersäure bewährt hat. Speziell werden die trockenen Trägerkörper einige Stunden (z.B. etwa 4 Stunden lang) in etwa 5%iger Salpetersäure gekocht, von der Säure getrennt, gewaschen, getrocknet und dann etwa einen Tag lang in einem Unterdruckraum mit ca. 3%iger DEAE-Dextran-Lösung bei Zimmertemperatur belassen oder nach Anlegen von Unterdruck autoklaviert. Schließlich werden die aus der Dextran-Lösung entnommenen Trägerkörper von überschüssiger Lösung befreit, getrocknet und sind dann unmittelbar oder nach Lagerung für die Immobilisierung von Mikroorganismen verwendbar.

Die vorteilhafte Wirkung der erfindungsgemäßen Beschichtung von anorganischen Trägerkörpern wird nachfolgend am Beispiel der L-Leucinproduktion mit Corynebacterium Glutamicum ATCC 13032 aus α-Ketoisocaproat anhand der beigefügten Kurvenbilder veranschaulicht; es zeigen:

Fig. 1 bis 3 die Leucinausbeute in Abhängigkeit von der Zeit bei Verwendung unterschiedlicher Trägerkörper;

Fig. 4 u. 6 bis 8 die Zahl der pro Gramm Träger fixierten Mikroorganismen und

Fig. 5 Die Zahl der pro Gramm Träger innerhalb unterschiedlicher Zeiten immobilisierten Mikroorganismen an aus unterschiedlich konzentrierten Lösungen erzielten DEAE-Dextran-Beschichtungen.

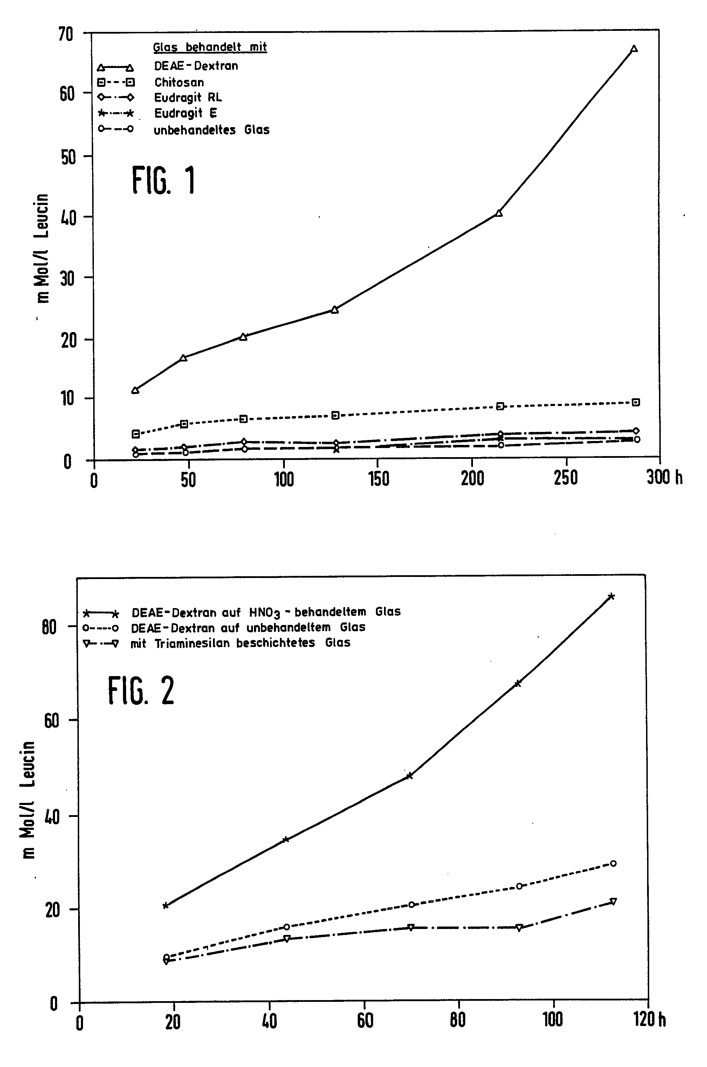

Die Fig. 1 - 3 zeigen Vergleichswerte für die L-Leucinproduktion aus α-Ketoisocaproat unter Verwendung C. Glutamicum ATCC 13032 unter jeweils vergleichbaren Bedingungen mit unterschiedlich behandelten Glasträgern von offenporigem Sinterglas gemäß der DE-OS 34 10 650. Wie aus Fig. 1 ersichtlich ist, wird durch DEAE-Dextranbeschichtung des Glases im Vergleich zu unbeschichtetem oder mit anderen Materialien einschließlich des vorbeschriebenen Chitosans beschichtetem Glas eine deutlich höhere Produktivität erzielt, die auf eine verbesserte Fixierung und Aktivität der fixierten Organismen zurückgeht.

Alle Gläser wurden vor der Beschichtung 3,5 Std. lang mit 5%iger Salpetersäurelösung gekocht, gründlich gewaschen und getrocknet.

Aus Fig. 2 sind Ergebnisse von Verleichsversuchen ersichtlich, die die vorteilhafte Wirkung der Vorbehandlung des Glases mit starker Säure vor der immobilisierungsfördernden Beschichtung zeigen.

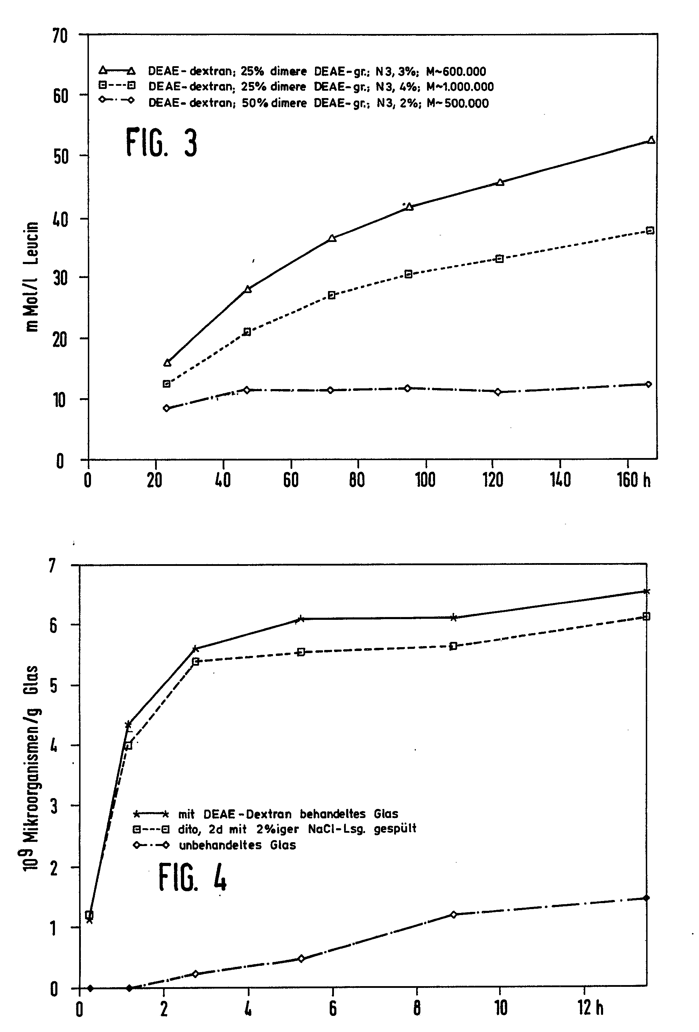

In Fig. 3 sind schließlich Vergleichsdaten aufgetragen, die mit DEA-dextran-beschichteten Trägerkörpern nach Vorbehandlung mit Salpetersäure erzielt wurden: wie man sieht, sind ein relativ geringer Dimeranteil, ein hoher Stickstoffgehalt und eine begrenzte Kettenlänge für die Immobilisierung produktiver Mikroorganismen förderlich.

In Fig. 4 ist die Zahl der an DEAE-dextran-beschichtetem Glas immobilisierten Mikroorganismen ($10^9$ Mikroorganismen pro Gramm Glas) gegen die Aufenthaltsdauer in einer Zellsuspension von C. Glutamicum ATCC 13032 in 2%iger NaCl-Lösung (entsprechend der üblichen Ionenstärke unter Fermentationsbedingungen) aufgetragen. Dabei wurden 20 g Borosilikat glasträger in 40 ml Zellsuspension mit ca. $2 \cdot 10^{10}$ Zellen/ml geschüttelt. Verglichen wurde unbehandeltes Glas mit DEAE-dextran-behandeltem Glas. Zusätzlich wurde DEAE-dextran-behandeltes Glas vorab zwei Tage lang mit 2%iger NaCl-Lösung gespült, um die Haftung der DEAE- Dextranadsorptionsschicht am Glas zu prüfen. Wie man sieht, ist die Fixierung von Mikroorganismen an dem vorab gespülten Glas nur unwesentlich verringert. Die Absolutwerte der Biomasse sind sehr hoch und erreichen fast dei den geometrischen Abmessungen entsprechende maximal mögliche Belegung des Trägers, was auch durch mikroskopische Untersuchungen verifiziert werden konnte.

Im praktischen Betrieb wurden kontinuierliche Fermentationen über fast drei Monate mit gleichbleibend guter Immobilisierung von Biomasse an den DEAE-dextran-beschichteten Trägern erfolgreich betrieben.

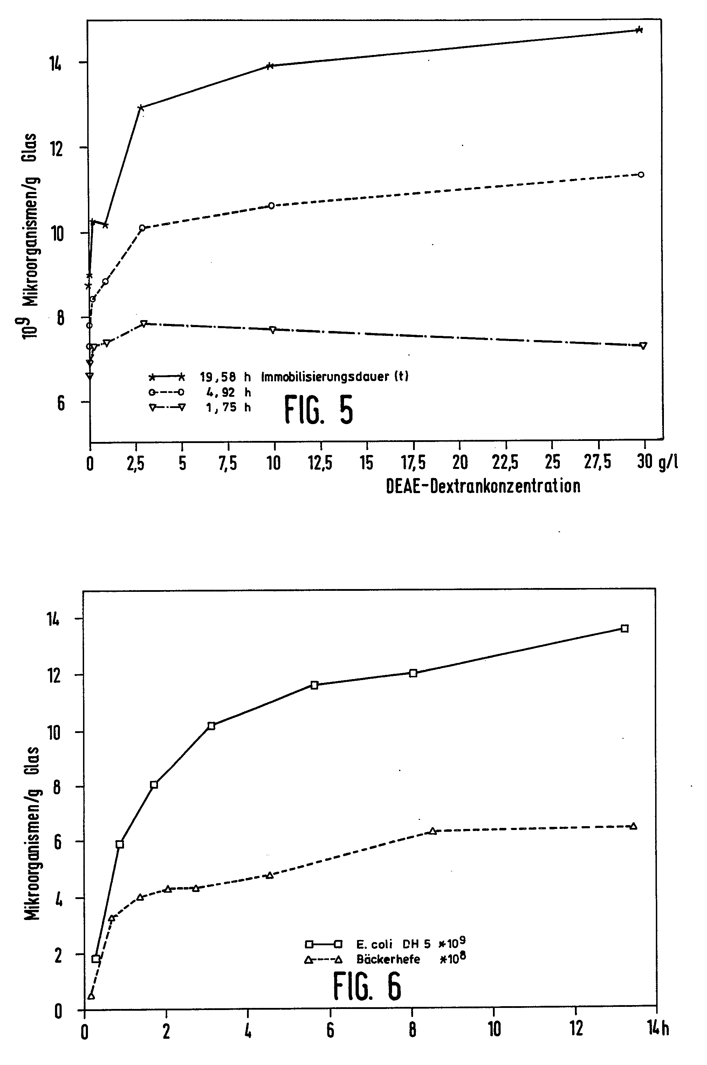

Fig. 5 veranschaulicht schließlich den Einfluß der DEAE-Dextrankonzentration der Lösung aus der die Glasbeschichtung durch Autoklavieren an säurevorbehandeltem Glas erfolgte, auf die Anzahl der angelagerten Mikroorganismen innerhalb unterschiedlicher Aufenthaltsdauer (t) der unterschiedlichen beschichteten Glasträger in einer Suspension von Mikroorganismen (mit jeweils ca. 1,6 $\cdot 10^{10}$ Zellen pro ml 2%iger NaCl-Lösung).

Wie man sieht, sollte die DEAE-Dextrankonzentration der Beschichtungslösung zumindest etwa 0,3%ig, vorzugsweise aber etwa 3%ig sein.

Von wesentlichem Einfluß auf die Zahl der aufgezogenen Mikroorganismen ist selbstverständlich die Aufenthaltsdauer in der Suspension derselben.

Vorstehend wurde die Nützlichkeit der Erfindung anhand von Untersuchungen mit Corynebacterium glutamicum dargelegt. Selbstverständlich ist die Erfindung nicht auf die Anwendung mit speziellen Bakterien beschränkt, wie die in Fig. 6 dargestellten Versuchsergebnisse mit Escherichia coli K12, Mutante DH5 (s. DNA cloning Vol. 1, Glover D. M. ed., IRL Press Oxford, 1985) und Bäckerhefe (Uniferm GmbH & Co., 4712 Werne, Charge 631228) zeigen:

Aufgetragen ist die Zahl der an DEAE-dextranbeschichtetem Glas immobilisierten Mikroorganismen gegen die Aufenthaltsdauer in der jeweiligen Zellsuspension in 2%iger NaCl-Lösung mit einer Anfangskonzentration von $1,1 \times 10^{10}$/ ml (E. coli) bzw. $4,3 \times 10^8$/ml (Bäckerhefe).

Für die Untersuchungen wurden jeweils 20 g der beschichteten Trägerkörper aus offenporigem Sinterglas gemäß der DE-OS 34 10 650 in 40 ml Zellsuspension geschüttelt. Wie man sieht, werden beide Mikroorganismen ebenfalls gut vom mit DEAE-Dextran beschichteten Trägerkörper aufgenommen. Die im Vergleich zu E. coli geringere Belegung des Glases mit Bäckerhefe-Organismen entspricht im wesentlichen den Größenverhältnissen der Zellen, so daß bei beiden Mikroorganismen von einer etwa vergleichbaren Oberflächendichte der am Glasträger adsorbierten Zellen ausgegangen werden kann.

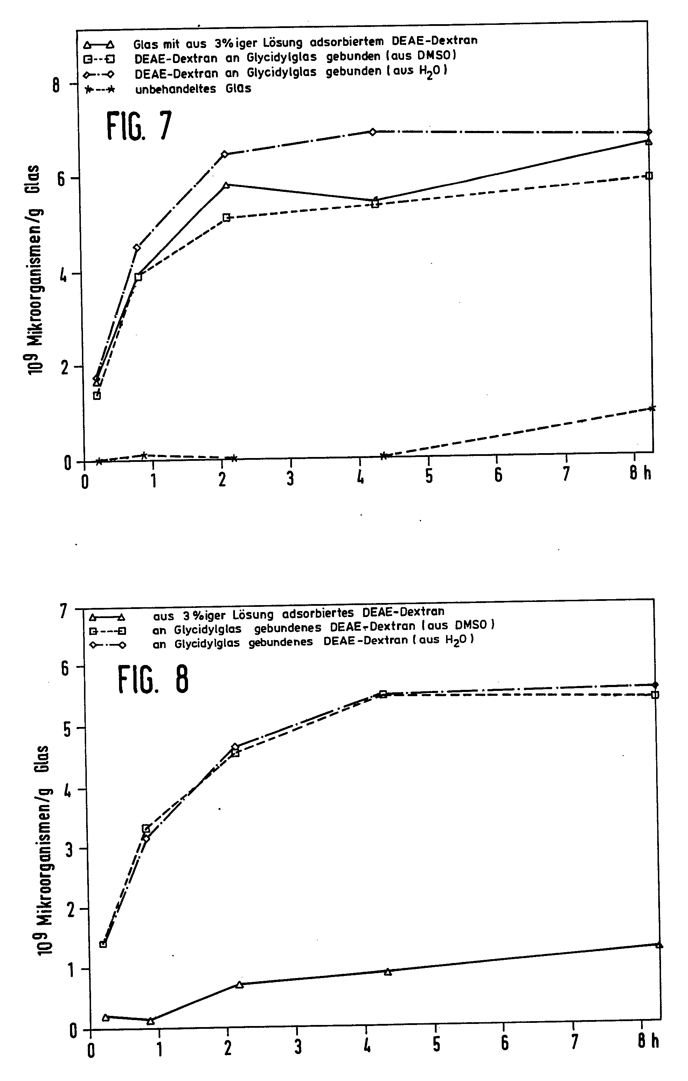

In Fig. 7 und 8 sind Vergleichswerte für die Immobilisierung von Corynebacterium Glutamicum ATCC 13032 unter jeweils vergleichbaren Bedingungen mit unterschiedlich behandelten Trägerkörpern von offenporigem Sinterglas gemäß der DE-OS 34 10 650 dargestellt:

Gewaschene Mikroorganismen wurden in 2%iger NaCl-Lösung suspendiert (jeweils etwa 2 x $10^{10}$ Zellen/ml) und je 40 ml dieser Suspension zu 20 g Trägerkörpern der jeweils genannten Spezifierung gegeben und bei Raumtemperatur geschüttelt.

Fig. 7 zeigt das Anlagerungsvermögen von Trägerkörpern, die adsorptiv bzw. kovalent mit DEAE-Dextran modifiziert waren. Die kovalente Kopplung von DEAE-Dextran erfolgte aus 3%iger Lösung einmal in wäßriger Phase und einmal in wasserfreiem Dimethylsulfoxid an mit Glycidylsilan behandeltem Glas. Wie man sieht, ist die Immobilisierungsfähigkeit der drei mit DEAE-Dextran behandelten Gläser in etwa gleich und deutlich besser als von unbehandelten Gläsern.

Fig. 8 veranschaulicht die überlegenen Eigenschaften der kovalent modifizierten Trägerkörper gegenüber dem nur adsorptiv modifizierten Trägerkörper. Hierbei wurde das Material vor dem Immobilisierungstest eine Woche lang mit 1 m $Na_2CO_3$ von pH 11,5 gespült.

Alle Prozentangaben bedeuten Gew.% mit Ausnahme der Angaben für die dimeren DEAE-Gruppen, die sich auf die Gesamtmenge der im DEAE-Dextran vorhandenen DEAE-Gruppen beziehen.

## Ansprüche

1. Anorganische Trägerkörper, insbesondere aus Glas oder Keramik, mit basischer, aminhaltiger Oberflächenschicht zur Immobilisierung von Mikroorganismen oder von menschlichen, tierischen oder pflanzlichen Zellen,
**dadurch gekennzeichnet,**
daß die Oberflächenschicht durch Dialkylaminoalkyl-Dextran, insbesondere DEAE-Dextran, gebildet wird und an den anorganischen Trägerkörper adsorptiv oder insbesondere kovalent gebunden ist.

2. Trägerkörper nach Anspruch 1,
**dadurch gekennzeichnet,**
daß die Oberflächenschicht durch DEAE-Dextran mit einem Anteil an dimeren DEAE-Gruppen von < 50 %, insbesondere ≤ 35 % und einem Molekulargewicht von 100 000 bis 2 x $10^6$, insbesondere bis $10^6$ bei einem N-Gehalt von 0,5 bis 5 %, insbesondere 3 bis 4 % gebildet wird.

3. Trägerkörper nach Anspruch 2,
**dadurch gekennzeichnet,**
daß die Oberflächenschicht durch DEAE-Dextran mit einem Anteil an dimeren DEAE-Gruppen von etwa 25 % und einem Molekulargewicht um 600 000 bei einem N-Gehalt von 3,3 % gebildet wird.

4. Trägerkörper nach einem der vorangehenden Ansprüche,
**dadurch gekennzeichnet,**
daß die Oberflächenschicht kovalent an einen Hydroxygruppen aufweisenden Glas- oder Keramikkörper gebunden ist.

5. Trägerkörper nach Anspruch 4,
**dadurch gekennzeichnet,**
daß die Oberflächensicht an eine Epoxyalkylglas- oder Chloralkylglasoberfläche oder durch Diisocyanat an eine Aminoalkylglasoberfläche kovalent gebunden ist.

6. Trägerkorper nach einem der vorangehenden Ansprüche,
**dadurch gekennzeichnet,**
daß die Oberflächenschicht auf den Oberflächen eines offenporigen Sinterglasträgers vorgesehen ist.

7. Trägerkörper nach Anspruch 6,
**gekennzeichnet durch**
eine Porendoppelstruktur des Sinterglases mit porositätsbestimmenden durchgehenden Makroporen von 20 - 500 μm Durchmesser und die Makroporenwände durchsetzenden Mikroporen von 1 - 10 μm Durchmesser.

8. Verfahren zur Herstellung von Trägerkörpern nach Anspruch 1 mit adsorptiv gebundener Dialkylaminoalkyl(DAAA) dextranschicht,
**dadurch gekennzeichnet,**
daß man die bzw. den anorganischen Trägerkörper großer Oberfläche zumindest 1 h in ≥ 5%iger starker Säure kocht und nach Auswaschen und Trocknen ≥ 1 %ige DAAA-Dextranlösung unter vermindertem Druck in die inneren Hohlräume eindringen läßt und schließlich die aus der Lösung entnommenen Trägerkörper von überschüssiger Lösung befreit und trocknet.

9. Verfahren nach Anspruch 8,
**dadurch gekennzeichnet,**
daß man die Trägerkörper etwa 4 h lang in ~5%iger Salpetersäure kocht.

10. Verfahren nach anspruch 8 oder 9,
**dadurch gekennzeichnet,**
daß man die mit Säure behandelten, gewaschenen und getrockneten Trägerkörper zur Benetzung der Hohlräume mit DAAA-Dextranlösung etwa 1 d lang unter Unterdruck in ca. 3%iger DAAA-Dextranlösung bei Zimmertemperatur beläßt oder in der Lösung nach einem Entgasen durch Anlegen von Unterdruck insbesondere 20 min lang bei 120°C und 1 atü autoklaviert.

11. Verwendung der Trägerkörper nach einem der Ansprüche 1 bis 7 für mit Mikroorganismen oder menschlichen, tierischen oder pflanzlichen Zellen ablaufende biotechnologische Prozesse.

FIG. 1

Glas behandelt mit
- DEAE-Dextran
- Chitosan
- Eudragit RL
- Eudragit E
- unbehandeltes Glas

- mMol/l Leucin

FIG. 2

- DEAE-Dextran auf $HNO_3$ - behandeltem Glas
- DEAE-Dextran auf unbehandeltem Glas
- mit Triaminesilan beschichtetes Glas

mMol/l Leucin

FIG. 3

DEAE-dextran; 25% dimere DEAE-gr.; N3, 3%; M~600.000
DEAE-dextran; 25% dimere DEAE-gr.; N3, 4%; M~1.000.000
DEAE-dextran; 50% dimere DEAE-gr.; N3, 2%; M~500.000

m Mol/l Leucin

FIG. 4

mit DEAE-Dextran behandeltes Glas
dito, 2d mit 2%iger NaCl-Lsg. gespült
unbehandeltes Glas

$10^9$ Mikroorganismen/g Glas

FIG. 5

FIG. 6

FIG. 7

△—△ Glas mit aus 3%iger Lösung adsorbiertem DEAE-Dextran
◻--◻ DEAE-Dextran an Glycidylglas gebunden (aus DMSO)
◇-·-◇ DEAE-Dextran an Glycidylglas gebunden (aus $H_2O$)
✳--✳ unbehandeltes Glas

$10^9$ Mikroorganismen/g Glas

FIG. 8

△—△ aus 3%iger Lösung adsorbiertes DEAE-Dextran
◻--◻ an Glycidylglas gebundenes DEAE-Dextran (aus DMSO)
◇-·-◇ an Glycidylglas gebundenes DEAE-Dextran (aus $H_2O$)

$10^9$ Mikroorganismen/g Glas